Europäisches Patentamt
European Patent Office
Office européen des brevets

(19)

(11) Publication number: **0 244 088**
**A2**

(12) **EUROPEAN PATENT APPLICATION**

(21) Application number: **87302724.7**

(22) Date of filing: **30.03.87**

(51) Int. Cl.⁴: **C07D 223/16** , A61K 31/55

The title of the invention has been amended (Guidelines for Examination in the EPO, A-III, 7.3).

(30) Priority: **03.04.86 US 847976**

(43) Date of publication of application: **04.11.87 Bulletin 87/45**

(84) Designated Contracting States: **AT BE CH DE ES FR GB GR IT LI LU NL SE**

(71) Applicant: **SMITHKLINE BECKMAN CORPORATION**
**One Franklin Plaza P O Box 7929**
**Philadelphia Pennsylvania 19103(US)**

(72) Inventor: **Bondinell, William Edward**
**1512 Franklin Lane**
**Wayne Pennsylvania 19087(US)**
Inventor: **Ormsbee III, Herbert Stowell**
**652 Pugh Road**
**Wayne Pennsylvania 19087(US)**

(74) Representative: **Waters, David Martin, Dr. et al**
**Smith Kline & French Laboratories Ltd.**
**Patent Department Mundells**
**Welwyn Garden City Hertfordshire AL7 1EY(GB)**

# (54) 1-Phenyl-3-benzazepine compounds and medicaments containing these compounds for treating gastrointestinal motility disorders.

(57) Compounds of the formula (I)

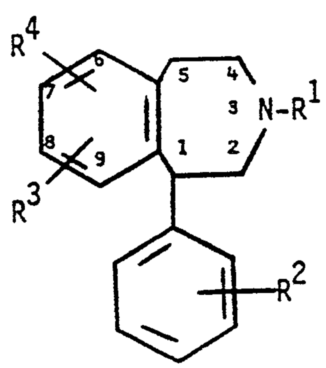

(I)

and pharmaceutically acceptable acid addition salts thereof for use in treating gastrointestinal motility disorders are described wherein $R^1$ is hydrogen, $C_1$-$C_6$alkyl or $C_3$-$C_5$alkenyl; $R^2$ is hydrogen, hydroxy, $C_1$-$C_6$alkoxy, halogen, trifluoromethyl, $C_1$-$C_6$alkyl, $SO_n(C_1$-$C_6)$alkyl, $SO_nCF_3$, $SO_n$phenyl or $SO_2NR^5R^6$; $R^3$ is hydrogen, halogen, $CF_3$, $C_1$-$C_6$alkyl or $R^7O$; $R^4$ is hydroxy, $C_1$-$C_6$alkoxy, $C_1$-$C_6$alkyl, $SO_n(C_1$-$C_6)$ alkyl, $NH_2$, $NO_2$, halogen, trifluoromethyl, $C_1$-$C_6$alkylsulfonamido, $C_1$-$C_6$alkanoylamino, hydroxymethyl or $C_1$-$C_6$alkoxymethyl, except that when $R^3$ is hydroxy in the 7-position and $R^1$ is hydrogen or when $R^3$ is hydroxy in the 8-position and $R^1$ is methyl, $R^4$ may be halogen or trifluoromethyl only if it is in the 6-or 9-position; n is 0, l or 2; $R^5$ and $R^6$ are hydrogen or $C_1$-$C_6$alkyl; and $R^7$ is hydrogen, $C_1$-$C_6$alkyl or $C_1$-$C_6$alkanoyl.

EP 0 244 088 A2

Pharmaceutical compositions containing them and their use in the manufacture of medicaments for treating gastrointestinal motility disorders are described.

## METHODS OF TREATING GASTROINTESTINAL MOTILITY DISORDERS

This invention relates to I-phenyl-3-benzazepine compounds for use in treating gastrointestinal motility disorders, pharmaceutical compositions containing them, and their use in the manufacture of medicaments for treating gastrointestinal motility disorders.

These compounds are known from U.S. Patent Nos. 3,393,I92; 4,327,023; 4.284,556; European Patent Applications 84/00245; 79,200,209 and U.K. Patent No. I,268,243. Some of the compounds have been reported as anti-bacterials, diuretics, anti-depressants, anti-parkinsonian agents, anti-hypertensives and analgesics, whilst for others there has been no disclosure of any pharmacological activity.

It has now been found that the I-phenyl-3-benzazepine compounds are useful therapeutically for gastroesophageal reflux disease and disorders of delayed gastric emptying of various etiologies including diabetes, surgery, and idiopathic delayed emptying. They may also be useful in treating disorders of upper GI motility, aspiration, early satiety, anorexia nervosa, and in diagnostic radiology or to facilitate intubation.

According to the present invention there is provided the use of a compound of the formula (I) :

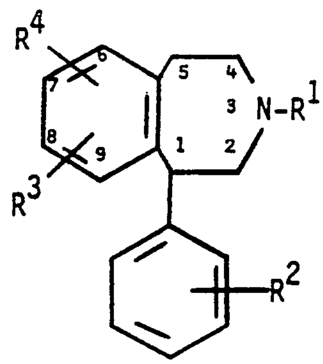

(I)

in which:

$R^1$ is hydrogen, $C_1$-$C_6$alkyl or $C_3$-$C_5$alkenyl;

$R^2$ is hydrogen, hydroxy, $C_1$-$C_6$alkoxy, halogen, trifluoromethyl, $C_1$-$C_6$alkyl, $SO_n(C_1$-$C_6)$alkyl, $SO_nCF_3$, $SO_n$ phenyl or $SO_2NR^5R^6$;

$R^3$ is hydrogen, halogen, $CF_3$, $C_1$-$C_6$alkyl or $R^7O$;

$R^4$ is hydroxy, $C_1$-$C_6$alkoxy, $C_1$-$C_6$alkyl, $SO_n(C_1$-$C_6)$alkyl, $NH_2$, $NO_2$, halogen, trifluoromethyl, $C_1$-$C_6$alkylsulfonamido, $C_1$-$C_6$alkanoylamino, hydroxymethyl or $C_1$-$C_6$alkoxymethyl, except that when $R^3$ is hydroxy in the 7-position and $R^1$ is hydrogen or when $R^3$ is hydroxy in the 8-position and $R^1$ is methyl, $R^4$ may be halogen or trifluoromethyl only if it is in the 6-or 9-position;

n is 0, I or 2;

$R^5$ and $R^6$ are hydrogen or $C_1$-$C_6$alkyl; and

$R^7$ is hydrogen, $C_1$-$C_6$alkyl or $C_1$-$C_6$alkanoyl;

or a pharmaceutically acceptable acid addition salt thereof, in the manufacture of a medicament for the treatment of gastrointestinal motility disorders.

Preferably, $R^2$ is hydroxy, $SO_2CH_3$, $SOCH_3$, $SO_2NH_2$, $SO_2NHCH_3$, or $SO_2N(CH_3)_2$.

Particular compounds of formula (I) are those in which $R^4$ is in the 7-position and those in which $R^4$ and $R^3$ are in the 7 and 8-positions respectively.

A group of compounds of formula (I) is that in which $R^4$ is $SO_n(C_1$-$C_6)$alkyl, particularly $SO_2$methyl; $R^3$ is methoxy or hydroxy; $R^1$ is hydrogen or methyl; and $R^2$ is hydroxy, $SO_2CH_3$, $SOCH_3$, $SO_2NH_2$, $SO_2NHCH_3$ or $SO_2N(CH_3)_2$, and, in addition, $R^4$ is in the 7-position and $R^3$ is in the 8-position.

Specific compounds of formula (I) are:

8-hydroxy-7-methylsulfonyl-I-phenyl-2,3,4,5-tetrahydro-IH-3-benzazepine;

8-hydroxy-I-(4-hydroxyphenyl)-7-methyl-2,3,4,5-tetrahydro-IH-3-benzazepine;

7,8-dihydroxy-I-phenyl-2,3,4,5-tetrahydro-IH-3-benzazepine;

7-hydroxy-8-methylsulfonyl-I-phenyl-2,3,4,5-tetrahydro-IH-3-benzazepine;

8-methoxy-7-methylsulfonyl-I-phenyl-2,3,4,5-tetrahydro-IH-3-benzazepine;

8-hydroxy-7-methyl-I-phenyl-2,3,4,5-tetrahydro-IH-3-benzazepine;

7-bromo-8-methoxy-3-methyl-I-phenyl-2,3,4,5-tetrahydro-IH-3-benzazepine

7-bromo-8-hydroxy-I-phenyl-2,3,4,5-tetrahydro-IH-3-benzazepine;

6-chloro-8-hydroxy-l-phenyl-2,3,4,5-tetrahdro-lH-3-benzazepine;
7-amino-8-hydroxy-l-phenyl-2,3,4,5-tetrahydro-lH-3-benzazepine;
7,8-dichloro-3-methyl-l-methyl-l-phenyl-2,3,4,5-tetrahydro-lH-3-benzazepine;
8-hydroxy-l-phenyl-7-trifluoromethyl-2,3,4,5-tetrahydro-lH-3-benzazepine;
8-hydroxy-7-methylthio-l-phenyl-2,3,4,5-tetrahydro-lH-3-benzazepine;
8-hydroxy-7-methoxymethyl-l-phenyl-2,3,4,5-tetrahydro-lH-3-benzazepine;
8-hydroxy-7-hydroxymethyl-l-phenyl-2,3,4,5-tetrahydro-lH-3-benzazepine:
7-hydroxy-8-methanesulfonamido-l-phenyl-2,3,4,5-tetrahydro-lH-3-benzazepine;
7-acetamido-8-hydroxy-l-phenyl-2,3,4,5-tetrahydrolH-3-benzazepine.

The compounds of formula (I) are optically active and exist as racemates and as (R) and (S) enantiomers. Resolution of the optical isomers may be accomplished by standard procedures such as fractional crystallization of their salts with optically active acids from appropriate solvents. The compounds used in the methods and pharmaceutical compositions of this invention include all isomers whether separated or mixtures thereof.

In another aspect the present invention provides a compound of the formula (II):

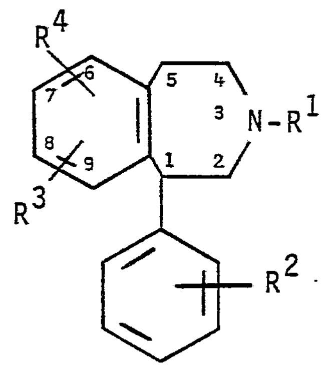

(II)

in which:

$R^1$ is hydrogen, $C_1$-$C_6$alkyl or $C_3$-$C_5$alkenyl;

$R^2$ is hydrogen, hydroxy, $C_1$-$C_6$alkoxy, halogen, trifluoromethyl, $C_1$-$C_6$alkyl, $SO_n$ ($C_1$-$C_6$)alkyl, $SO_nCF_3$, $SO_n$phenyl or $SO_2NR^5R^6$;

$R^3$ is hydrogen, halogen, $CF_3$, $C_1$-$C_6$alkyl or $R^7O$;

$R^4$ is SO($C_1$-$C_6$)alkyl, $SO_2$($C_1$-$C_6$)alkyl; $C_2$-$C_6$alkylsulfonamido, formamido, $C_3$-$C_6$alkanoylamino, hydroxymethyl or $C_1$-$C_6$alkoxymethyl;

n is 0, l, or 2;

$R^5$ and $R^6$ are hydrogen or $C_1$-$C_6$alkyl; and

$R^7$ is hydrogen, $C_1$-$C_6$alkyl or $C_1$-$C_6$alkanoyl; or a pharmaceutically acceptable acid addition salt thereof, for use as a therapeutic agent.

Specific compounds for use in this invention are :

8-hydroxy-7-methylsulfonyl-l-phenyl-2,3,4,5-tetrahydro-lH-3-benzazepine,
7-hydroxy-8-methylsulfonyl-l-phenyl-2,3,4,5-tetrahydro-lH-3-benzazepine,
8-methoxy-7-methylsulfonyl-l-phenyl-2,3,4,5-tetrahydro-lH-3-benzazepine.
8-hydroxy-7-methoxymethyl-l-phenyl-2,3,4,5-tetrahydro-lH-3-benzazepine, or
8-hydroxy-7-hydroxymethyl-l-phenyl-2,3,4,5-tetrahydro-lH-3-benzazepine

and pharmaceutically acceptable acid addition salts thereof.

The compounds of formula (I) are known from U.S. Patent Nos. 3,393,l92; 4,327,023; 4.284.556: European Patent Applications 8400245; 79200209 and U.K. Patent No. l,268.243. and may be prepared as follows:

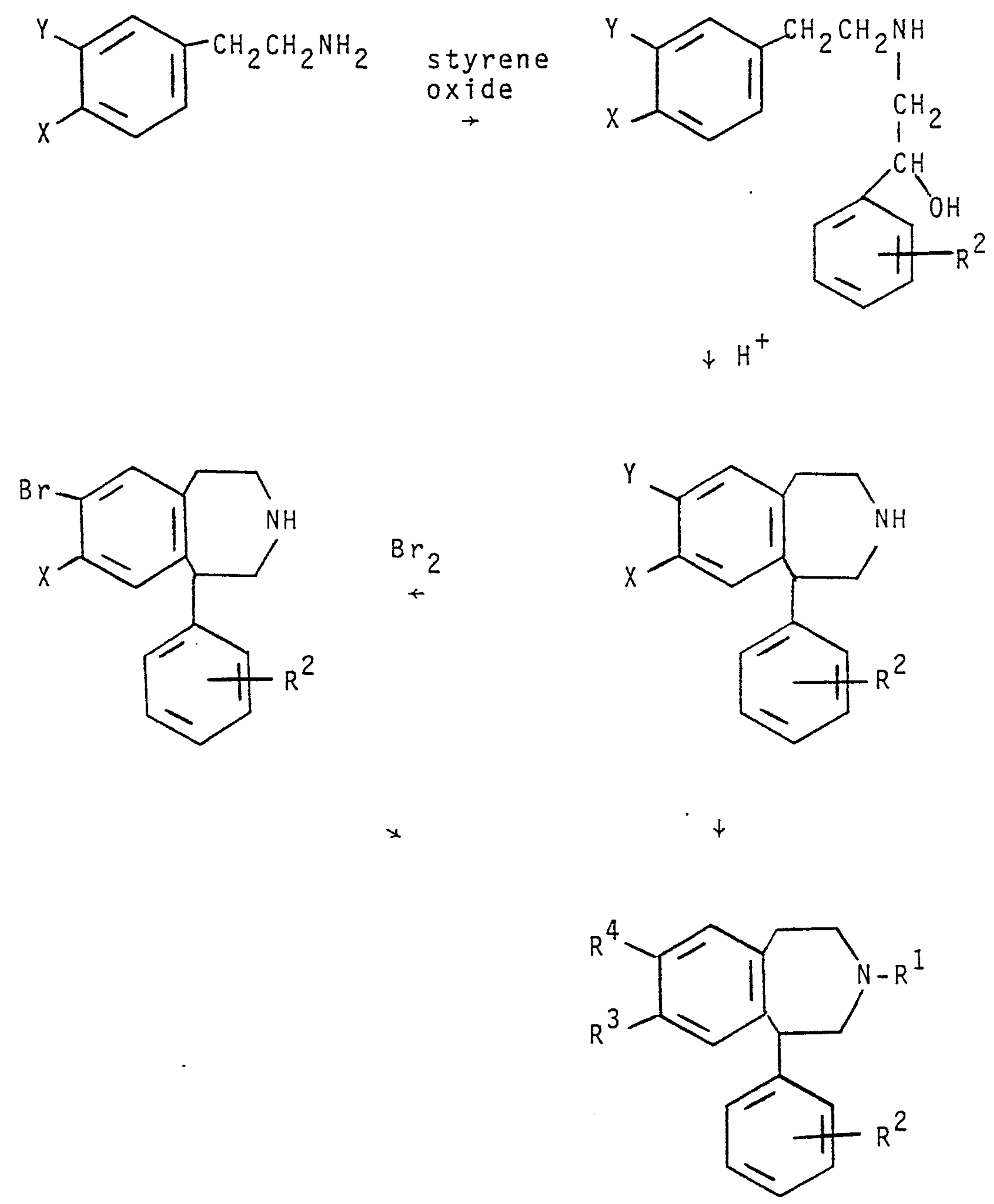


X is hydrogen or alkoxy or hydroxy
Y is hydrogen or alkoxy or hydroxy

According to the above procedure, an appropriately substituted or unsubstituted phenethylamine is reacted with an appropriately substituted styrene oxide and the resulting N-[l-(2-hydroxy-2-phenyl)ethyl]-phenethylamine is cyclized with acid to give a l-phenyl-2,3,4,5-tetrahydro-lH-3-benzazepine. If desired, a salt, for example the hydrochloride, of this compound may be reacted with bromine to give a bromo substituted benzazepine. The bromo substituent may be converted to other $R^4$ groups by standard procedures. These compounds may be N-alkylated, O-deprotected, O-alkylated or O-alkanoylated by standard methods and may be carried out in various order of steps to prepare the desired compounds.

Alternatively, the l-phenyl-2,3,4,5-tetrahydro-lH-3-benzazepine compound is N-acylated, then reacted with chlorosulfonic acid to give the chlorosulfonyl compound which is converted by standard procedures to lower alkylthio (and alkylsulfonyl and alkylsulfinyl) compounds of formula (I).

The hydroxy compounds are prepared by O-demethylating the methoxy compounds by standard procedures. The compounds are optionally O-alkylated, O-alkanoylated, N-alkylated or N-alkenylated. These procedures are carried out by standard methods and may be carried out in various order of steps to prepare the desired compounds.

The compounds of formula (I) form pharmaceutically acceptable acid addition salts with organic or inorganic acids. Examples of these acids are hydrochloric, hydrobromic, sulfuric, phosphoric, acetic, tartaric, citric, maleic, lactic, oxalic, succinic, methanesulfonic, and benzenesulfonic acids. The salts are formed according to methods known to the art. If the product is isolated as an acid addtion salt, it may be treated with an inorganic or organic base, such as aqueous sodium hydroxide, sodium, carbonate, triethylamine, etc., and converted to the corresponding free base. The base can then be treated with an appropriate acid, for example in an aqueous miscible solvent, such as a lower alkanol preferably methanol or ethanol, to give the desired salt.

The effect of the pharmacologically active compounds of formula (I) on gastrointestinal motility is demonstrated in test procedures as follows:

(I) an increase in resting pressure of the lower esophageal sphincter (LES) in dogs: and

(2) an increase in the rate of gastric emptying in rats.

Method for Determination of LES Pressure in the Anesthetized Dog

Mongrel or beagle dogs, male and female, are anesthetized using sodium pentobarbital (35.0 mg/kg.. I.V.). Sodium pentobarbital is then continuously infused (approximatemated 6.0 mg/kg/hr) to maintain deep anesthesia. Blood pressure is monitored via a catheter surgically implanted into the femoral artery and attached to a Gould's P231D transducer. A catheter is also implanted into the femoral vein to administer test drugs. Respiration is maintained by an endotracheal tube attached to a respirator. A continuously perfused manometric catheter system including a Dent sleeve to measure sphincter pressure (Dent, Gastroenterology 7I: 263-267, I976) is inserted into the esophagus and positioned so that intraluminal pressure is recorded from the body of the esophagus, the lower esophageal sphincter (LES) and the fundus of the stomach. The Dent sleeve catheter is perfused at a rate of 0.5 ml of water per minute for each lumen of the catheter by using an Arndorfer Hydraulic Capillary Infusion System. A cannula is implanted into the gastric antrum to allow drainage of the perfusate solution and prevent intestinal distension. Continuous tracings of esophageal, LES and fundus pressure are monitored on a Grass Polygraph (Model 7D). Correct positioning of the Dent sleeve is verified by noting a high pressure zone at the LES and by administration of an intravenous dose of 5-HT (usually I0-I5 mcg/kg) which contracts the LES while having little or no recordable effect on either the body of the esophagus or the fundus. After verification of placement of the sleeve, the animal is allowed to stabilize for approximately 30 minutes.

Compounds are administered intravenously, intraduodenally or intragastrically. In most cases, succeeding doses of the same or different compounds are given only after the LES pressure has returned to approximate predosing baseline values. In all cases, the magnitude of change in LES pressure is determined from the baseline pressure immediately prior to each treatment to the maximum pressure during treatment. Since the compounds used usually produce an immediate effect on LES pressure, no pretreatment time prior to measurement is necessary during this testing.

Direct assay techniques are used to estimate an Effective Dose 20 ($ED_{20}$) for the test compound in individual animals. The mean $ED_{20}$ and 95% confidence limits are determined using the individual $ED_{20}$ values from a group of animals (N = ≧3) that received the same treatment. Table I gives the $ED_{20}$ for certain compounds of this invention.

## Table 1

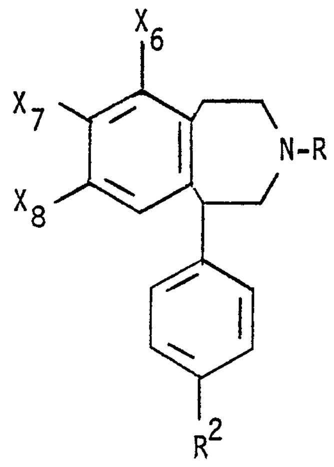

| Cpd. No. | $X_6$ | $X_7$ | $X_8$ | R | $R^2$ | $ED_{20}$ mcg/kg, i.v. |
|---|---|---|---|---|---|---|
| 1 | Cl | H | OH | H | H | 445 |
| 2 | H | OH | $CH_3SO_2$ | H | H | 317 |
| 3 | H | $CH_3SO_2$ | OH | H | H | 50 |
| 4 | H | Br | OH | H | H | 16 |
| 5 | H | $CH_3$ | OH | H | H | 26 |
| 6 | H | $CH_3S$ | OH | H | H | 17 |
| 7 | H | $CF_3$ | OH | H | H | 20 |
| 8 | H | Br | $CH_3O$ | $CH_3$ | H | 79 |
| 9 | H | Cl | Cl | $CH_3$ | H | 623 |
| 10 | H | OH | OH | H | H | 735 |
| 11 | H | $CH_3$ | OH | H | OH | 44 |
| 12 | H | $CH_3SO_2$ | $CH_3O$ | H | H | 15 mm Hg at 250 (i.g.) |
| 13 | H | $CH_3OCH_2$ | OH | H | H | <50 |
| 14 | H | $HOCH_2$ | OH | H | H | 100 |
| 15 | H | OH | $CH_3SO_2NH$ | | H | 500 |
| 16 | H | $CH_3CONH$ | OH | H | H | < 2000 |

Gastric Emptying in the Rat

Fasted rats are administered 0.5 µCi of $Na_2CrO_4$ (0.2 ml vol) into the stomach with an oral feeding tube. Compounds for evaluation or vehicle controls are administered either I5 minutes before (oral administration) or simultaneously with (intravenous administration) the test meal. After 35 minutes the rats are killed by cervical dislocation and the stomach is removed. Gastric emptying is measured from the amount of $^{51}Cr$ remaining in the stomach at death. The rate of gastric emptying as compared to control is determined.

The method of treating gastrointestinal motility disorders in accordance with this invention comprises administering internally to a subject in need of said treatment an effective amount of a compound of formula (I) or a pharmaceutically acceptable acid addition salt thereof. The compound will preferably be administered in a dosage unit form orally or parenterally. Advantageously, equal doses will be administered one to four times daily with the daily dosage regimen being from about I mg. to about I000 mg., preferably from 2 mg. to 400 mg. the method described above is useful for treating gastrointestinal motility disorders.

Preferably, the compounds of formula (I) are administered in conventional dosage unit forms prepared by combining an appropriate dose of the compound with standard pharmaceutical carriers. The dosage units will contain the active ingredient in an effective amount selected from about 0.25 mg. to about 250 mg., preferably 0.5 mg. to I00 mg.

The pharmaceutical carrier employed may be, for example, either a solid or liquid. Exemplary of solid carriers are lactose, terra alba, sucrose, talc, gelatin, agar, pectin, acacia, magnesium stearate, stearic acid and the like. Exemplary of liquid carriers are syrup, peanut oil, olive oil, water and the like. Similarly, the carrier or diluent can include any time delay material well known to the art, such as glyceryl monostearate or glyceryl distearate alone or with a wax.

A wide variety of pharmaceutical forms can be employed. Thus, if a solid carrier is used, the preparation can be tableted, placed in hard gelatin capsule in powder or pellet form or in the form of a trouche or lozenge. The amount of solid carrier will vary widely but preferably will be from about 25 mg. to about I g. If a liquid carrier is used, the preparation will be in the form of a syrup, emulsion, soft gelatin capsule, sterile injectable liquid such as an ampul or an aqueous or nonaqueous liquid suspension.

The pharmaceutical compositions are prepared by conventional techniques involving procedures such as mixing, granulating and compressing when necessary or variously mixing and dissolving the ingredients as appropriate to the desired composition.

One skilled in the art will recognize that in determining the amounts of the compound needed to produce the desired pharmacological effect without toxic side effects, the activity of the particular compound as well as the size of the host animal must be considered.

This invention also includes pharmaceutical compositions for treatment of gastrointestinal motility disorders comprising a pharmaceutically acceptable carrier and a compound of the formula (II) as hereinbefore defined or a pharmaceutically acceptable acid addition salt thereof.

The following examples illustrate the invention but are not to be construed as limiting the scope thereof. Temperatures are in degrees Centigrade unless otherwise stated.

EXAMPLE I

8-Methoxy-I-phenyl-2,3,4,5-tetrahydro-IH-3-benzazepine.

4-Methoxyphenethylamine (I48 g, 0.98 m) and styrene oxide (II8 g, 0.98 m) were stirred and heated to 95° for I6 hours. The mixture was poured into 3:I hexane-ethyl acetate (600 ml), diluted with hexane and the resulting solid was obtained by filtration to give N-[I-(2-hydroxy-2-phenyl)ethyl]-4-methoxyphenethylamine.

The amine (I06 g, 0.39 m) was refluxed in a mixture of trifluoroacetic acid (I.2 l) and concentrated sulfuric acid (30 ml, 0.57 m) for 2 hours. The trifluoroacetic acid was evaporated in vacuo and the residue poured into ice water (I.2 l). The solution was made alkaline with 40% aqueous sodium hydroxide and extracted with ethyl acetate. The ethyl acetate solution was acidified with hydrogen chloride gas to give 8-methoxy-I-phenyl-2,3,4,5-tetrahydro-IH-3-benzazepine hydrochloride, m.p. 230-233°.

EXAMPLE 2

7-Bromo-8-methoxy-l-phenyl-2,3,4,5-tetrahydro-lH-3-benzazepine.

8-Methoxy-l-phenyl-2,3,4,5-tetrahydro-lH-3-benzazepine hydrochloride (66 g, 0.23 m) was suspended in acetic acid (300 ml) and treated with bromine (43 g, 0.27 m). The mixture was heated to 95° for one hour, cooled and filtered. The filter cake was partitioned between ethyl acetate and ammonium hydroxide to afford 7-bromo-8-methoxy-l-phenyl-2,3,4,5-tetrahydro-lH-3-benzazepine which was converted to the hydrochloride salt, m.p. 225-227°.

EXAMPLE 3

8-Methoxy-7-methylsulfonyl-l-phenyl-2,3,4,5-tetrahydro-lH-3-benzazepine; 8-Hydroxy-7-methylsulfonyl-l-phenyl-2,3,4,5-tetrahydro-lH-3-benzazepine; 8-methoxy-7-methylthio-l-phenyl-2,3,4,5-tetrahydro-lH-3-benzazepine; 8-hydroxy-7-methylthio-l-phenyl-2,3,4,5-tetrahydro-lH-3-benzazepine.

Method A

7-Bromo-8-methoxy-l-phenyl-2,3,4,5-tetrahydro-lH-3-benzazepine hydrochloride (l0 g, 0.027 m) was converted to the free base by partitioning between chloroform and aqueous sodium hydroxide. A solution of the free base (5.3 g, 0.0l6 m) in toluene (50 ml) was added to a solution of N-butyllithium (0.07 m) in toluene (25 ml) stirred at -78°. The mixture was stirred for thirty minutes, treated with a solution of methyl disulfide (l3 g, 0.l4 m) in toluene (20 ml), stirred for fifteen minutes and poured into water (200 ml). The mixture was acidified with l0% hydrochloric acid and the aqueous phase was washed with ether, made alkaline with aqueous sodium hydroxide and extracted with ethyl acetate. The combined ethyl acetate extracts were washed, dried with sodium sulfate and concentrated in vacuo. The residue was dissolved in ether and treated with ethereal HCl to give 8-methoxy-7-methylthio-l-phenyl-2,3,4,5-tetrahydro-lH-3-benzazepine hydrochloride, m.p. l95-200° after recrystallization from acetone-isopropanol.

8-Methoxy-7-methylthio-l-phenyl-2,3,4,5-tetrahydro-lH-3-benzazepine hydrochloride was refluxed with 48% hydrobromic acid and concentrated in vacuo to afford 8-hydroxy-7-methylthio-l-phenyl-2,3,4,5-tetrahydro-lH-3-benzazepine hydrobromide, m.p. 207-2ll°.

A mixture of 8-methoxy-7-methylthio-l-phenyl-2,3,4,5-tetrahydro-lH-3-benzazepine (2 g, 0.007 m) and trifluoroacetic anhydride (l.5 g, 0.007 m) in methylene chloride (40 ml) was stirred for l hour, treated with methanol and filtered to afford 8-methoxy-7-methylthio-l-phenyl-3-trifluoroacetyl-2,3,4,5-tetrahydro-lH-3-benzazepine.

The trifluoracetamide (2.6 g) was suspended in glacial acetic acid (75 ml), treated with 30% hydrogen peroxide (20 ml), and stirred for l6 hours. The mixture was diluted with water (500 ml) and extracted with ether. The combined ether extracts were washed with l0% aqueous sodium bicarbonate and water, dried with sodium sulfate and concentrated in vacuo to afford 8-methoxy-7-methylsulfonyl-l-phenyl-3-trifluoracetyl-2,3,4,5-tetrahydro-lH-3-benzazepine.

The trifluoroacetamide (l.7 g, 0.004 m) was dissolved in methanol (40 ml) containing 40% sodium hydroxide and stirred for l6 hours. The methanol was evaporated and the residue dissolved in ethyl acetate which was washed, dried with sodium sulfate and concentrated in vacuo to afford 8-methoxy-7-methylsulfonyl-l-phenyl-2,3,4,5-tetrahydro-lH-3-benzazepine.

8-Methoxy-7-methylsulfonyl-l-phenyl-2,3,4,5-tetrahydro-lH-3-benzazepine (l.2 g, 0.004 m) in 3.5 ml of methylene chloride was cooled to -l5° and treated with boron tribromide (0.0l4 m) to afford 8-hydroxy-7-methylsulfonyl-l-phenyl-2,3,4,5-tetrahydro-lH-3-benzazepine hydrobromide, m.p. 240-242° (d). This is treated with ammonium hydroxide. The resulting mixture is filtered to give 8-hydroxy-7-methylsulfonyl-2,3,4,5-tetrahydro-lH-3-benzazepine. This is treated with methanesulfonic acid to give the methanesulfonate.

Method B

8-Methoxy-l-phenyl-2,3,4,5-tetrahydro-lH-3-benzazepine hydrochloride (28.9 g, 0.l mol) and anhydrous sodium acetate (8.2 g, 0.l mol) were stirred in acetic anhydride (l25 ml) for l6 hours and concentrated in in vacuo. Toluene was added and the mixture was concentrated in vacuo to remove traces of acetic anhydride. The residue was partitioned between methylene chloride and 5% aqueous sodium bicarbonate. The methylene chloride phase was dried with sodium sulfate and concentrated in vacuo to give 28.5 g (96%) of crude 3-acetyl-8-methoxy-l-phenyl-2,3,4,5-tetrahydro-lH-3-benzazepine.

3-Acetyl-8-methoxy-l-phenyl-2,3,4,5-tetrahydro-lH-3-benzazepine (28.5 g, 0.096 mol) was dissolved in methylene chloride (500 ml). The solution was stirred and cooled to an internal temperature of -20°C with a Dry Ice/acetone bath. Chlorosulfonic acid (22.4 g, 0.l93 mol) in methylene chloride (50 ml) was added dropwise over 90 min with the internal temperature maintained between -20° to -25°C. The mixture was stirred at -20°C for an additional l5 min. The acetone bath was removed and the mixture was stirred at 25°C for l6 hours. The intermediate sulfonic acid precipitated from solution. The methylene chloride was evaporated in vacuo. Chloroform (500 ml) was added and the mixture was treated with excess thionyl chloride (l00 ml) and heated to reflux for 7 hours. The mixture was concentrated in vacuo and carbon tetrachloride was added and evaporated invacuo to remove traces of thionyl chloride. The reside was partitioned between methylene chloride and water and the methylene chloride phase was dried with sodium sulfate and concentrated in vacuo to give 43 g of crude 3-acetyl-7-chlorosulfonyl-8-methoxy-l-phenyl-2,3,4,5-tetrahydro-lH-3-benzazepine.

The crude product was purified by chromatography to afford 23 g (6l%) of 3-acetyl-7-chlorosulfonyl-8-methoxy-l-phenyl-2,3,4,5-tetrahydro-lH-3-benzazepine.

3-Acetyl-7-chlorosulfonyl-8-methoxy-l-phenyl-2,3,4,5-tetrahydro-lH-3-benzazepine (22.5 g, 0.057 mol) was dissolved in methylene chloride (50 ml) and added dropwise to a stirred mixture of sodium sulfite (8.95 g, 0.07l mol) and sodium bicarbonate (9.9 g, 0.ll8 mol) in water (48 ml) maintained at 70°C. The methylene chloride was allowed to distill from the reaction. The mixture was stirred for l5 min and treated with iodomethane (l6.4 g, 0.ll4 mol) and methanol (48 ml). The mixture was stirred at 70°C for l5 min, concentrated in vacuo and partitioned between methylene chloride and water. The methylene chloride phase was dried with sodium sulfate and concentrated in vacuo to give l6.5 g (77%) of 3-acetyl-8-methoxy-7-methylsulfonyl-l-phenyl-2,3,4,5-tetrahydro-lH-3-benzazepine.

3-Acetyl-8-methoxy-7-methylsufonyl-l-phenyl-2,3,4,5-tetrahydro-lH-3-benzazepine (l6.5 g, 0.044 mol) was suspended in a mixture of concentrated hydrochloric acid (64 ml) and water (l60 ml), stirred and heated to reflux for l6 hours. the mixture was concentrated in vacuo and stirred with acetone to give l3.9 g (86%) of 8-methoxy-7-methylsulfonyl-l-phenyl-2,3,4,5-tetrahydro-lH-3-benzazepine hydrochloride: m.p. > 240°C. A sample was recrystallized from methanol-ether: m.p. 25l-253°C.

## EXAMPLE 4

8-Hydroxy-7-methylsulfinyl-l-phenyl-2,3,4,5-tetrahydro-lH-3-benzazepine.

8-Methoxy-7-methylthio-l-phenyl-3-trifluoroacetyl-2,3,4,5-tetrahydro-lH-3-benzazepine is reacted with one equivalent of 30% hydrogen peroxide to afford 8-methoxy-7-methylsulfinyl-l-phenyl-3-trifluoroacetyl-2,3,4,5-tetrahydro-lH-3-benzazepine. The latter is heated with base to give 8-methoxy-7-methylsulfinyl-l-phenyl-2,3,4,5-tetrahydro-lH-3-benzazepine. Treating this 8-methoxy compound with boron tribromide by the procedure of Example 3 gives 8-hydroxy-7-methylsulfinyl-l-phenyl-2,3,4,5-tetrahydro-lH-3-benzazepine hydrobromide.

EXAMPLE 5

7-Bromo-8-methoxy-3-methyl-l-phenyl-2,3,4,5-tetrahydro-lH-3-benzazepine; 8-Hydroxy-3-methyl-7-methylthio-l-phenyl-2,3,4,5-tetrahydro-lH-3-benzazepine.

7-Bromo-8-methoxy-l-phenyl-2,3,4,5-tetrahydro-lH-3-benzazepine (9.6 g, 0.03 m), 37% formalin (27 ml) and 95% formic acid (38 ml) were mixed and heated to reflux for l6 hours. The mixture was concentrated in vacuo, treated with l0% hydrochloric acid (70 ml) and concentrated in vacuo. The residue was partitioned between ethyl acetate and ammonium hydroxide to give 7-bromo-8-methoxy-3-methyl-l-phenyl-2,3,4,5-tetrahydro-lH-3-benzazepine.

By the procedure of Example 3 Method A, 7-bromo-8-methoxy-3-methyl-l-phenyl-2,3.4.5-tetrahydro-lH-3-benzazepine is reacted with N-butyllithium and methyl disulfide and then with ethereal HCl to give 3-methyl-8-methoxy-7-methyl-thio-l-phenyl-2,3,4,5-tetrahydro-lH-3-benzazepine hydrochloride. This salt is refluxed with 48% hydrobromic acid by the procedure of Example 3 Method A to give 8-hydroxy-3-methyl-7-methylthio-l-phenyl-2,3,4,5-tetrahydro-lH-3-benzazepine hydrobromide.

EXAMPLE 6

By the procedures of Examples 3 Method A and 4, 3-methyl-8-methoxy-7-methylthio-l-phenyl-2,3,4,5-tetrahydro-lH-3-benzazepine (prepared as in Example 5) is oxidized to give:

3-methyl-8-methoxy-7-methylsulfonyl-l-phenyl-2,3,4,5-tetrahydro-lH-3-benzazepine and
3-methyl-8-methoxy-7-methylsulfinyl-l-phenyl-2,3,4,5-tetrahydro-lH-3-benzazepine.

These 8-methoxy compounds are treated with boron tribromide by the procedure of Example 3 Method A to give

8-hydroxy-3-methyl-7-methylsulfonyl-l-phenyl-2,3,4,5-tetrahydro-lH-3-benzazepine hydrobromide and
8-hydroxy-3-methyl-7-methylsulfinyl-l-phenyl-2,3,4,5-tetrahydro-lH-3-benzazepine hydrobromide.

EXAMPLE 7

8-Hydroxy-7-methylsulfonyl-l-[4-(methylsulfonyl)phenyl]-2,3,4,5-tetrahydro-lH-3-benzazepine.

3-Acetyl-8-methoxy-l-phenyl-2,3,4,5-tetrahydro-lH-3-benzazepine (5 g, 0.0l7 m) was added to chlorosulfonic acid (l2 ml) stirred at 5°. The mixture was stirred at 25° for l6 hours, poured carefully into ice water and extracted with methylene chloride. The methylene chloride phase was washed with water, dried with magnesium sulfate and concentrated in vacuo to afford 3-acetyl-7-chlorosulfonyl-l-[4-(chlorosulfonyl)phenyl]-8-methoxy-2,3,4,5-tetrahydro-lH-3-benzazepine.

Following the general procedure of Example 3 Method B, this compound was treated with sodium sulfite and sodium bicarbonate and then with iodomethane to give 3-acetyl-8-methoxy-7-methylsulfonyl-l-[4-(methylsulfonyl)-phenyl]-2,3,4,5-tetrahydro-lH-3-benzazepine.

Treating the 8-methoxy compound with hydrobromic acid by the procedure of Example 5 gave 8-hydroxy-7-methylsulfonyl-l-[4-(methylsulfonyl)phenyl]-2,3,4,5-tetrahydro-lH-3-benzazepine hydrobromide, m.p. 340° (d).

EXAMPLE 8

8-Hydroxy-l-(4-hydroxyphenyl)-7-methyl-2,3,4,5-tetrahydro-lH-3-benzazepine.

A mixture of 4-methoxy-3-methylphenethylamine (ll g, 0.066 m) and methyl 4-methoxymandelate (l3.2 g, 0.067 m) was heated to 95° for l6 hours to give N-[2-(4-methoxy-3-methylphenyl)ethyl]-4-methoxymandelamide, m.p. 7l-72°.

The mandelamide (l3 g, 0.04 m) was added to a solution of Vitride (0.l6 m) in toluene (250 ml), stirred 30 minutes at 25° and stirred for 90 minutes at reflux. The mixture was cooled and quenched with l0% aqueous sodium hydroxide (ll0 ml). The toluene phase was washed with water and brine, dried with sodium sulfate and concentrated in vacuo to give N-[l-(2-hydroxy-2-(4-methoxyphenyl))ethyl]-4-methoxy-3-methylphenethylamine, m.p. ll9-l20° (chloroform-ether).

Following the general procedure of Example I, the phenethylamine was treated with trifluoroacetic acid and sulfuric acid to give 8-methoxy-7-methyl-I-(4-methoxyphenyl)-2,3,4,5-tetrahydro-IH-3-benzazepine.

Following the general procedure of Example 3, the dimethoxy compound was reacted with boron tribromide to give 8-hydroxy-I-(4-hydroxyphenyl)-7-methyl-2,3,4,5-tetrahydro-IH-3-benzazepine hydrobromide, m.p. 280-284°.

## EXAMPLE 9

7-Methoxy-8-methylsulfonyl-I-phenyl-2,3,4,5-tetrahydro-IH-3-benzazepine: 7-Hydroxy-8-methylsulfonyl-I-phenyl-2,3,4,5-tetrahydro-IH-3-benzazepine.

A mixture of 3-methoxyphenylethylamine (6.I g, 0.04 m) and styrene oxide (4.9 g, 0.04 m) was stirred and heated at 95° for I6 hours. The mixture was treated with n-butyl chloride to give N-[I-(2-hydroxy-2-phenyl)ethyl]-3-methoxyphenylethylamine, m.p. 86-88°.

N-[I-(2-Hydroxy-2-phenyl)ethyl]-3-methoxyphenylethylamine (2 g, 7 mmol) dissolved in trifluoroacetic acid (20 ml) and concentrated sulfuric acid (2 ml) was refluxed for 2 hours and concentrated in vacuo. The residue was basified with I0% aqueous sodium hydroxide and extracted into ethyl acetate. The ethyl acetate was washed with water and with brine, dried with magnesium sulfate and concentrated in vacuo. The residue was dissolved in acetonitrile and treated with maleic acid to afford 7-methoxy-I-phenyl-2,3,4,5-tetrahydro-IH-3-benzazepine maleate, m.p. I65-I67°.

Following the general procedure of Example 2, 7-methoxy-I-phenyl-2,3,4,5-tetrahydro-IH-3-benzazepine maleate (9.6 g, 0.026 m) was reacted with bromine (8.3 g, 0.052 m) to afford 8-bromo-7-methoxy-I-phenyl-2,3,4,5-tetrahydro-IH-3-benzazepine. This bromo compound was treated with n-butyllithium and then with methyl disulfide to afford 7-methoxy-8-methylthio-I-phenyl-2,3,4,5-tetrahydro-IH-3-benzazepine hydrochloride which was converted to the free base.

Following the general procedure of Example 3 Method A, 7-methoxy-8-methylthio-I-phenyl-2,3,4,5-tetrahydro-IH-3-benzazepine was reacted with 30% hydrogen peroxide to give 7-methoxy-8-methylsulfonyl-I-phenyl-2,3,4,5-tetrahydro-IH-3-benzazepine.

Treating this 7-methoxy compound with 48% hydrobromic acid by the procedure of Example 3 Method A gave 7-hydroxy-8-methylsulfonyl-I-phenyl-2,3,4,5-tetrahydro-IH-3-benzazepine hydrobromide, m.p. 274-276°.

## EXAMPLE I0

8-Hydroxy-7-methyl-I-phenyl-2,3,4,5-tetrahydro-IH-3-benzazepine.

A mixture of 4-methoxy-3-methylbenzaldehyde (33 g, 0.22 m), nitromethane (40 g, 0.65 m) and ammonium acetate (I6.6 g, 0.22 m) in acetic acid (200ml) was heated for I6 hours, concentrated in vacuo, diluted with water and filtered. The filter cake was crystallized from toluene-hexane to afford 4-methoxy-3-methyl-(2-nitroethenyl)-benzene, m.p. 76-79°.

A solution of 4-methoxy-3-methyl-(2-nitroethenyl)benzene (32 g, 0.I65 m) in tetrahydrofuran (600 ml) was added to a solution of lithium aluminium hydride (0.435 m) in tetrahydrofuran (I85 ml), stirred for 20 minutes, cooled to 5° and carefully quenched with water (I6.5 ml), I0% aqueous sodium hydroxide (25 ml) and water (4I ml). The mixture was filtered and concentrated in vacuo. The residue was partitioned between ether and dilute hydrochloric acid. The aqueous phase was made alkaline with I0% aqueous sodium hydroxide and extracted with ether. The ether extract was washed, dried with magnesium sulfate and concentrated in vacuo. The residue was distilled in vacuo to afford 4-methoxy-3-methylphenethylamine, b.p. 90-98° (0.07 mm Hg).

By the general procedure of Example I, 4-methoxy-3-methylphenethylamine was reacted with styrene oxide and the resulting N-[I-(2-hydroxy-2-phenyl)ethyl]-4-methoxy-3-methylphenethylamine was cyclized to give 8-methoxy-7-methyl-I-phenyl-2,3,4,5-tetrahydro-IH-3-benzazepine hydrochloride, m.p. I95-200°. The hydrochloride was converted to the free base and refluxed with 48% hydrobromic acid to give 8-hydroxy-7-methyl-I-phenyl-2,3,4,5-tetrahydro-IH-3-benzazepine hydrobromide, m.p. 263-265°.

EXAMPLE 11

6-Chloro-8-hydroxy-l-phenyl-2,3,4,5-tetrahydro-lH-3-benzazepine.

Following the general procedure of Example 9, 2-chloro-4-methoxyphenethylamine was reacted with styrene oxide to afford N-[l-(2-hydroxy-2-phenyl)ethyl]-2-chloro-4-methoxyphenethylamine.

A solution of the phenethylamine (2 g, 0.007 m) in trifluoroacetic acid (90 ml) was added to a refluxing mixture of trifluoroacetic acid (430 ml) and trifluoromethanesulfonic acid (28 g). The mixture was cooled, concentrated in vacuo and the residue partitioned between methylene chloride and aqueous sodium hydroxide. The methylene chloride phase was washed, dried with sodium sulfate and concentrated in vacuo. The residue was converted to the hydrochloride to afford 6-chloro-8-methoxy-l-phenyl-2,3,4,5-tetrahydro-lH-3-benzazepine hydrochloride, m.p. 243-246°.

Following the general procedure of Example 3 Method A, 6-chloro-8-methoxy-l-phenyl-2,3,4,5-tetrahydro-lH-3-benzazepine hydrochloride (l.2 g, 0.004 m) was reacted with boron tribromide (0.008 m) to afford 6-chloro-8-hydroxy-l-phenyl-2,3,4,5-tetrahydro-lH-3-benzazepine hydrochloride. m.p. 278-282° (d).

EXAMPLE 12

7-Bromo-8-hydroxy-l-phenyl-2,3,4,5-tetrahydro-lH-3-benzazepine.

Following the general procedure of Example 3 Method A, 7-bromo-8-methoxy-l-phenyl-2,3,4,5-tetrahydro-lH-3-benzazepine (prepared as in Example 2) was refluxed with 48% hydrobromic acid to afford 7-bromo-8-hydroxy-l-phenyl-2,3,4,5-tetrahydro-lH-3-benzazepine hydrobromide, m.p. 2l8-220°.

EXAMPLE 13

8-Hydroxy-l-phenyl-7-trifluoromethyl-2,3,4,5-tetrahydro-lH-3-benzazepine.

A mixture of 7-bromo-8-methoxy-l-phenyl-2,3,4,5-tetrahydro-lH-3-benzazepine (3.3 g, 0.0l ml) and trifluoroacetic anhydride (2.l g, 0.0l m) in methylene chloride (50 ml) was stirred for l hour, treated with methanol and concentrated in vacuo to afford 7-bromo-8-methoxy-l-phenyl-3-trifluoroacetyl-2,3,4,5-tetrahydro-lH-3-benzazepine.

The benzazepine was reacted with trifluoromethyl iodide and activated copper as described by D. Gaitanopoulos and M. Brenner, Synthetic Communications l0(3), 2l3-2l9 (l980), to give 8-methoxy-l-phenyl-3-trifluoroacetyl-7-trifluoromethyl-2,3,4,5-tetrahydro-lH-3-benzazepine. This compound was deacylated and demethylated by the procedure described in Example 3 Method A to give 8-hydroxy-l-phenyl-7-trifluoromethyl-2,3,4,5-tetrahydro-lH-3-benzazepine hydrochloride, m.p. 223-225°.

EXAMPLE 14

7-Methylsulfonyl-l-phenyl-2,3,4,5-tetrahydro-lH-3-benzazepine.

Following the general procedure of Example l, 3-(methylthio)phenethylamine is reacted with styrene oxide and then with trifluoroacetic acid and sulfuric acid to give 7-methylthio-l-phenyl-2,3,4,5-tetrahydro-lH-3-benzazepine.

Following the general procedure of Example 3 Method A, the methylthio-benzazepine is treated with trifluoroacetic anhydride, hydrogen peroxide and finally with aqueous sodium hydroxide to give 7-methylsulfonyl-l-phenyl-2,3,4,5-tetrahydro-lH-3-benzazepine.

EXAMPLE I5

3-Allyl-8-hydroxy-7-methylsulfonyl-I-phenyl-2,3,4,5-tetrahydro-IH-3-benzazepine.

Allyl bromide (0.0I m) is added to a mixture of 8-hydroxy-7-methylsulfonyl-I-phenyl-2,3,4,5-tetrahydro-IH-3-benzazepine (0.0I m) and potassium carbonate (2 g) stirred in dry acetone (20 ml) at 5°. The mixture is stirred at 5°, then at 25° and is finally stirred at reflux. The mixture is poured into water. extracted with ethyl acetate and treated with ethereal hydrogen chloride to give 3-allyl-8-hydroxy-7-methylsulfonyl-I-phenyl-2,3,4,5-tetrahydro-IH-3-benzazepine hydrochloride.

EXAMPLE I6

8-Acetoxy-7-methylsulfonyl-I-phenyl-2,3,4,5-tetrahydro-IH-3-benzazepine.

A mixture of 8-hydroxy-7-methylsulfonyl-I-phenyl-2,3,4,5-tetrahydro-IH-3-benzazepine hydrobromide (4.0 g) in 25 ml. of trifluoroacetic acid is stirred at 25° and treated with I.4 g. of acetyl bromide. The mixture is stirred at 25° and concentrated in vacuo to give 8-acetoxy-7-methylsulfonyl-I-phenyl-2,3,4,5-tetrahydro-IH-3-benzazepine hydrobromide.

EXAMPLE I7

8-Hydroxy-7-methylsulfonyl-I-(4-sulfamoylphenyl)-2,3,4,5-tetrahydro-IH-3-benzazepine.

8-Methoxy-7-methylsulfonyl-I-phenyl-2,3,4,5-tetrahydro-IH-3-benzazepine (3.3 g, 0.0I mol) is stirred in a mixture of acetic anhydride and sodium acetate to afford 3-acetyl-8-methoxy-7-methylsulfonyl-I-phenyl-2,3,4,5-tetrahydro-IH-3-benzazepine. the acetyl compound (3.7 g, 0.0I mol) is treated with chlorosulfonic acid in methylene chloride to afford 3-acetyl-8-methoxy-7-methylsulfonyl-I-(4-chlorosulfonylphenyl)-2,3,4,5-tetrahydro-IH-3-benzazepine. The chlorosulfonyl compound (4.7 g, 0.0I mol) is dissolved in tetrahydrofuran and treated with ammonia to afford 3-acetyl-8-methoxy-7-methylsulfonyl-2-(4-sulfamoylphenyl)-2,3,4,5-tetrahydro-IH-3-benzazepine. The sulfamoyl compound (4.5 g, 0.0I mol) is refluxed in 3N hydrochloric acid to afford 8-methoxy-7-methylsulfonyl-I-(4-sulfamoylphenyl)-2,3,4,5-tetrahydro-IH-3-benzazepine hydrochloride. The hydrochloride (4.5 g, 0.0I mol) is treated with excess boron tribromide in methylene chloride to afford 8-hydroxy-7-methylsulfonyl-I-(4-sulfamoylphenyl)-2,3,4,5-tetrahydro-IH-3-benzazepine hydrobromide.

EXAMPLE I8

8-Hydroxy-7-methylsulfonyl-I-[4-(N-methylsulfamoyl)phenyl]-2,3,4,5-tetrahydro-IH-3-benzazepine.

Following the general procedure of Example I7, 3-acetyl-8-methoxy-7-methylsulfonyl-I-(4-chlorosulfonylphenyl)-2,3,4,5-tetrahydro-IH-3-benzazepine is treated with methylamine and then with hydrochloric acid to afford 8-methoxy-7-methylsulfonyl-I-[4-(N-methylsulfamoyl)phenyl]-2,3,4,5-tetrahydro-IH-3-benzazepine hydrochloride. This hydrochloride is treated with boron tribromide to give 8-hydroxy-7-methylsulfonyl-I-[4-(N-methylsulfamyl)phenyl]-2,3,4,5-tetrahydro-IH-3-benzazepine hydrobromide.

EXAMPLE I9

8-Hydroxy-7-methylsulfonyl-I-[4-(N-N-dimethylsulfamoyl)-phenyl]-2,3,4,5-tetrahydro-IH-3-benzazepine.

Following the general procedure of Example I7, 3-acetyl-8-methoxy-7-methylsulfonyl-I-(4-chlorosulfonylphenyl)-2,3,4,5-tetrahydro-IH-3-benzazepine is treated with dimethylamine and then with hydrochloric acid and the resulting compound is treated with boron tribromide to give 8-hydroxy-7-methylsulfonyl-I-[4-(N.N-dimethylsulfamoyl)phenyl]-2,3,4,5-tetrahydro-IH-3-benzazepine hydrobromide.

EXAMPLE 20

7-Hydroxy-l-(4-hydroxyphenyl)-8-methylsulfonyl-2,3,4,5-tetrahydro-lH-3-benzazepine; 8-hydroxy-l-(4-hydroxyphenyl)-7-methylsulfonyl-2,3,4,5-tetrahydro-lH-3-benzazepine.

4-Bromo-3-methoxybenzoic acid (23.l g, 0.l m) dissolved in tetrahydrofuran (200 ml) is added to lM borane in tetrahydrofuran (l50 ml) stirred at 5°. The mixture is refluxed, cooled, treated with methanol, refluxed and concentrated in vacuo to give 4-bromo-3-methoxybenzyl alcohol.

4-Bromo-3-methoxybenzyl alcohol (2l.7 g, 0.l m) dissolved in toluene (200 ml) containing pyridine (ll.8 g, 0.l5 m) is stirred at 5° and treated with thionyl chloride (23.8 g, 0.2 m). The mixture is stirred, diluted with water and the organic layer washed with dilute hydrochloric acid, dried and concentrated in vacuoto give 4-bromo-3-methoxybenzyl chloride.

4-Bromo-3-methoxybenzyl chloride (23.5 g, 0.l m) dissolved in ethanol (500 ml) is treated with potassium cyanide (l3 g, 0.2 m) dissolved in water. The mixture is warmed and stirred to give 4-bromo-3-methoxyphenylacetonitrile.

4-Bromo-3-methoxyphenylacetonitrile (22.6 g, 0.l m) dissolved in tetrahydrofuran (l00 ml) is added to lM borane in tetrahydrofuran (300 ml) stirred at 25°. The mixture is refluxed, cooled to 0° and treated cautiously with 2N hydrochloric acid to give 4-bromo-3-methoxyphenethylamine.

Following the general procedure of Example 8, 4-bromo-3-methoxyphenethylamine (2.3 g, 0.l m) is treated with methyl 4-methoxymandelate (l8 g, 0.l m) to give N-[l-[2-hydroxy-2-(4-methoxyphenyl)]ethyl]-4-bromo-3-methoxyphenethylamine. This N-substituted 4-bromo-3-methoxyphenethylamine (3.8 g, 0.0l m) is refluxed in a mixture of trifluoroacetic acid (20 ml) and sulfuric acid (20 ml) to give 8-bromo-7-methoxy-l-(4-methoxyphenyl)-2,3,4,5-tetrahydro-lH-3-benzazepine.

8-Bromo-7-methoxy-l-(4-methoxyphenyl)-2,3,4,5-tetrahydro-lH-3-benzazepine (3.6 g, 0.0l m) is reacted with n-butyllithium in ether and then with methyl disulfide to give 7-methoxy-l-(4-methoxyphenyl)-8-methylthio-2,3,4,5-tetrahydro-lH-3-benzazepine.

The methylthio compound (3.3. g, 0.0l m) is treated with trifluoroacetic anhydride to afford 7-methoxy-l-(4-methoxyphenyl)-8-methylthio-3-trifluoroacetyl-2,3,4,5-tetrahydro-lH-3-benzazepine. The trifluoroacetyl compound (4.3 g, 0.0l m) is dissolved in methylene chloride and treated with 3-chloroperoxybenzoic acid to afford 7-methoxy-l-(4-methoxyphenyl)-8-methylsulfonyl-3-trifluoroacetyl-2,3,4,5-tetrahydro-lH-3-benzazepine. This compound (4.6 g, 0.0l m) is stirred with aqueous sodium hydroxide to afford 7-methoxy-l-(4-methoxyphenyl)-8-methylsulfonyl-2,3,4,5-tetrahydro-lH-3-benzazepine. The 7-methoxy compound (3.5 g, 0.0l m) is treated with excess boron tribromide in methylene chloride to afford 7-hydroxy-l-(4-hydroxyphenyl)-8-methylsulfonyl-2,3,4,5-tetrahydro-lH-3-benzazepine hydrobromide.

By the same procedure, using 3-bromo-4-methoxybenzoic acid as the starting material, the product is 8-hydroxy-l-(4-hydroxyphenyl)-7-methylsulfonyl-2,3,4,5-tetrahydro-lH-3-benzazepine hydrobromide.

EXAMPLE 2l

8-Hydroxy-7-methylsulfonyl-l-[3-trifluoromethylsulfonyl)phenyl]-2,3,4,5-tetrahydro-lH-3-benzazepine.

Following the general procedure of Example l, 4-methoxyphenethylamine is reacted with 3-bromostyrene oxide to afford l-(3-bromophenyl)-8-methoxy-2,3,4,5-tetrahydro-lH-3-benzazepine.

Following the general procedure of Example 20, the bromo compound (3.3 g, 0.0l m) is treated with n-butyllithium followed by trifluoromethylsulfenyl chloride to afford 8-methoxy-l-[3-(trifluoromethylthio)phenyl]-2,3,4,5-tetrahydro-lH-3-benzazepine.

Following the general procedure of Example 7, the trifluoromethylthio compound (3.5 g, 0.0l m) is treated with acetic anhydride and then with two equivalents of chlorosulfonic acid followed by thionyl chloride in chloroform to afford 3-acetyl-7-chlorosulfonyl-8-methoxy-l-[3-(trifluoromethylthio)phenyl]-2,3,4,5-tetrahydro-lH-3-benzazepine.

By the general procedure of Example 3, Method B, the chlorosulfonyl compound (4.9 g, 0.0l m) is reduced with sodium sulfite and sodium bicarbonate, and treated with methyl iodide and then with hydrobromic acid to afford 8-hydroxy-7-methylsufonyl-l-[3-(trifluoromethylsulfonyl)phenyl]-2,3,4,5-tetrahydro-lH-3-benzazepine hydrobromide.

EXAMPLE 22

I-(4-Chlorophenyl)-8-hydroxy-7-methylsulfonyl-2,3,4,5-tetrahydro-IH-3-benzazepine.

Following the general procedure of Example I, 4-methoxyphenethylamine is reacted with 4-chlorostyrene oxide to afford I-(4-chlorophenyl)-8-methoxy-2,3,4,5-tetrahydro-IH-3-benzazepine which is then treated with acetic anhydride, chlorosulfonic acid and thionyl chloride by the procedures of Example 2I to afford 3-acetyl-I-(4-chlorophenyl)-7-chlorosulfonyl-8-methoxy-2,3,4,5-tetrahydro-IH-3-benzazepine.

Following the general procedure of Example 3, Method B, the chlorosulfonyl compound is treated with sodium sulfite and sodium bicarbonate, followed by iodomethane and then with hydrobromic acid to afford I-(4-chlorophenyl)-8-hydroxy-7-methylsulfonyl-2,3,4,5-tetrahydro-IH-3-benzazepine hydrobromide.

EXAMPLE 23

7-Acetamido-8-hydroxy-I-phenyl-2,3,4,5-tetrahydro-IH-3-benzazepine.

A mixture of 4.0 g. (0.0I0 mole) of 7-acetamido-3-benzyl-8-hydroxy-I-phenyl-2,3,4,5-tetrahydro-IH-3-benzazepine, prepared as in U.S. Patent No. 4,327,023, 0.57 ml. (0.0097 mole) of glacial acetic acid and I50 ml. of methanol-ethyl acetate was hydrogenated over 4.0 g. of I0% palladium-on-charcoal at 60 p.s.i. After I.5 hours, the mixture was flushed with argon and filtered. The filtrate was evaporated in vacuo at 45°-50° to give a yellow-green oily residue. The residue was dissolved in water. The aqueous solution was neutralized with sodium carbonate to give 2.7 g. (87%) of white solid 7-acetamido-8-hydroxy-I-phenyl-2,3,4,5-tetrahydro-IH-3-benzazepine, m.p. I33°-I43°.

EXAMPLE 24

7-Hydroxy-8-methylsulfonamido-I-phenyl-2,3,4,5-tetrahydro-IH-3-benzazepine.

A mixture of 6.2 g (0.0I43 mole) of 8-amino-3-benzyl-7-benzyloxy-I-phenyl-2,3,4,5-tetrahydro-IH-3-benzazepine, I.72 g (0.0I50 mole) of methanesulfonyl chloride. 30 ml. of triethylamine and 200 ml of benzene was stirred at room temperature overnight. Assay of the mixture indicated incomplete reaction. An additional 0.6 ml (0.0075 mole) of sulfonyl chloride was added and the reaction continued until completion. The reaction mixture was washed with alkali, dried and evaporated. The residual oil (8.I g) was dissolved in methane and seeded to give crystalline 3-benzyl-7-benzyloxy-8-methylsulfonamido-I-phenyl-2,3,4,5-tetrahydro-IH-3-benzazepine, m.p. I36°-I38°.

This intermediate (3.55 g, 0.00693 mole) was dissolved in methanol-ethanol (I:I) and hydrogenated over I0% palladium-on-charcoal in an acid medium. The mixture was flushed with argon. filtered and the filtrate evaporated. The residue was taken up in water and neutralized with sodium carbonate. The resulting solid was taken up in methanol. The filtrate was reacted with an excess of fumaric acid in methanol. After filtration ether is added to induce crystallization of I.0 g of 7-hydroxy-8-methylsulfonamido-I-phenyl-2,3,4,5-tetrahydro-IH-3-benzazepine hemifumarate hydrate, m.p. I78°-I82° (dec.).

EXAMPLE 25

7-Methoxymethyl-8-hydroxy-I-phenyl-2,3,4,5-tetrahydro-IH-3-benzazepine.

A solution of 7-bromo-8-methoxy-I-phenyl-2,3,4,5-tetrahydro-IH-3-benzazepine (7.5 g, 0.0225 m) in toluene (40 ml) was added to butyllithium (2.I m, 42 ml) stirred at 78°. The mixture was stirred for thirty minutes and poured into ether (200 ml) saturated with carbon dioxide. The mixture was acidified with hydrochloric acid, extracted with ether and the aqueous phase concentrated to give 7-carboxy-8-methoxy-I-phenyl-2,3,4,5-tetrahydro-IH-3-benzazepine hydrochloride.

7-Carboxy-8-methoxy-l-phenyl-2,3,4,5-tetrahydro-lH-3-benzazepine hydrochloride (l0.7 g) in 48% hydrobromic acid (l00 ml) was refluxed for 2.5 hours, cooled and filtered to give 7-carboxy-8-hydroxy-l-phenyl-2,3,4,5-tetrahydro-lH-3-benzazepine hydrobromide, m.p. 278-280°, which was dissolved in water, treated with methanol and then with sodium bicarbonate to give 7-carboxy-8-hydroxy-l-phenyl-2,3,4,5-tetrahydro-lH-3-benzazepine, m.p. 302-305°.

7-Carboxy-8-hydroxy-l-phenyl-2,3,4,5-tetrahydro-lH-3-benzazepine (2.8 g, 0.009 m) was added to diborane (0.04 m) in tetrahydrofuran (ll3 ml) stirred at 0°. The mixture was stirred at 0° for 2 hours, at 25° for l6 hours and at reflux for l hour. The mixture was treated with methanol (40 ml), refluxed for l hour and concentrated in vacuo. The residue was dissolved in methanol, treated with ethereal hydrogen chloride and concentrated in vacuo. The residue was chromatographed on silica eluted with chloroform-methanol and crystallized from ethyl acetate-methanol to give 7-methoxymethyl-8-hydroxy-l-phenyl-2,3,4,5-tetrahydro-lH-3-benzazepine hydrochloride, m.p. 300-307° (d).

EXAMPLE 26

7-Hydroxymethyl-8-hydroxy-l-phenyl-2,3,4,5-tetrahydro-lH-3-benzazepine.

Following the general procedure of Example 25, 7-carboxy-8-hydroxy-l-phenyl-2,3,4,5-tetrahydro-lH-3-benzazepine was treated with diborane, stirred at 25°, treated with ethanol and concentrated on a steam bath. The residue was treated with ethanol and concentrated hydrochloric acid and concentrated. The residue was treated with ethereal hydrogen chloride and crystallized from methanol-ethyl acetate-ether to give 7-hydroxymethyl-8-hydroxy-l-phenyl-2,3,4,5-tetrahydro-lH-3-benzazepine hydrochloride, m.p. 322-330° (d).

EXAMPLE 27

8-Hydroxy-7-methylsulfonyl-l-phenyl-2,3,4,5-tetrahydro-lH-3-benzazepine methanesulfonate (l0 mg) is mixed with 85 mg. of lactose and 3 mg. of magnesium stearate and filled into a hard gelatin capsule.

EXAMPLE 28

| Ingredients | Amounts |
|---|---|
| 8-Hydroxy-7-methylsulfonyl-1-phenyl-2,3,4,5-tetrahydro-1H-3-benzazepine methanesulfonate | 10 mg |
| Calcium sulfate dihydrate | 250 mg |
| Sucrose | 40 mg |
| Starch | 25 mg |
| Talc | 7 mg |
| Stearic acid | 5 mg |

The calcium sulfate dihydrate, sucrose and the benzazepine compound are thoroughly mixed and granulated with l0% gelatin solution. The wet granules are screened, dried and then mixed with the starch, talc and stearic acid, screened and compressed into a tablet.

Similarly, capsules and tablets using other compounds of formula (I) or (II) as active ingredients are prepared according to the above procedures.

## Claims

1. A compound of formula (II)

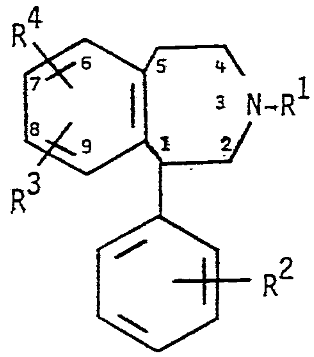

(II)

in which:
$R^1$ is hydrogen, $C_1$-$C_6$-alkyl or $C_3$-$C_5$alkenyl;
$R^2$ is hydrogen, hydroxy, $C_1$-$C_6$alkoxy, halogen, trifluoromethyl, $C_1$-$C_6$alkyl, $SO_n$ ($C_1$-$C_6$)alkyl, $SO_nCF_3$, $SO_n$phenyl or $SO_2NR^5R^6$;
$R^3$ is hydrogen, halogen, $CF_3$, $C_1$-$C_6$alkyl or $R^7O$;
$R^4$ is SO ($C_1$-$C_6$)alkyl, $SO_2$($C_1$-$C_6$)alkyl,
$C_2$-$C_6$alkylsulfonamido, formamido, $C_3$-$C_6$alkanoylamino, hydroxymethyl or $C_1$-$C_6$alkoxymethyl;
n is O, I or 2;
$R^5$ and $R^6$ are hydrogen or $C_1$-$C_6$alkyl; and
$R^7$ is hydrogen, $C_1$-$C_6$alkyl or $C_1$-$C_6$alkanoyl;
or a pharmaceutically acceptable acid addition salt thereof, for use as a therapeutic agent.

2. A compound of claim I which is 8-hydroxy-7-methylsulfonyl-I-phenyl-2,3,4,5-tetrahydro-IH-3-benzazepine.

3. A compound of claim I which is:

7-hydroxy-8-methylsulfonyl-I-phenyl-2,3,4,5-tetrahydro-IH-3-benzazepine,

8-methoxy-7-methylsulfonyl-I-phenyl-2,3,4,5-tetrahydro-IH-3-benzazepine,

8-hydroxy-7-methoxymethyl-I-phenyl-2,3,4,5-tetrahydro-IH-3-benzazepine, or

8-hydroxy-7-hydroxymethyl-I-phenyl-2,3,4,5-tetrahydro-IH-3-benzazepine.

or a pharmaceutically acceptable acid addition salt thereof.

4. A pharmaceutical composition comprising a pharmaceutical carrier and a compound of claim I, 2 or 3.

5. The use of a compound of formula (I)

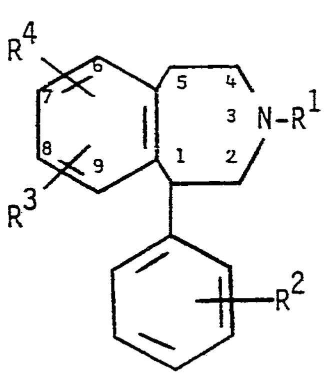

(I)

in which:

$R^1$ is hydrogen, $C_1$-$C_6$alkyl or $C_3$-$C_5$alkenyl;

$R^2$ is hydrogen, hydroxy, $C_1$-$C_6$alkoxy, halogen, trifluoromethyl, $C_1$-$C_6$alkyl, $SO_n(C_1$-$C_6)$alkyl, $SO_nCF_3$, $SO_n$phenyl or $SO_2NR^5R^6$;

$R^3$ is hydrogen, halogen, $CF_3$, $C_1$-$C_6$alkyl or $R^7O$;

$R^4$ is hydroxy, $C_1$-$C_6$alkoxy, $C_1$-$C_6$alkyl, $SO_n(C_1$-$C_6)$ alkyl, $NH_2$, $NO_2$, halogen, trifluoromethyl, $C_1$-$C_6$alkylsulfonamido, $C_1$-$C_6$alkanoylamino, hydroxymethyl or $C_1$-$C_6$-alkoxymethyl, except that when $R^3$ is hydroxy in the 7-position and $R^1$ is hydrogen or when $R^3$ is hydroxy in the 8-position and $R^1$ is methyl, $R^4$ may be halogen or trifluoromethyl only if it is in the 6-or 9-position;

n is 0, I or 2;

$R^5$ and $R^6$ are hydrogen or $C_1$-$C_6$alkyl; and

$R^7$ is hydrogen, $C_1$-$C_6$alkyl or $C_1$-$C_6$alkanoyl;

or a pharmaceutically acceptable acid addition salt thereof; in the manufacture of a medicament for the treatment of gastrointestinal motility disorders.

6. The use of a compound of formula I as defined in claim 5 in which in formula (I) $R^2$ is hydroxy, $SO_2CH_3$, $SOCH_3$, $SO_2NH_2$, $SO_2NHCH_3$ or $SO_2N(CH_3)_2$.

7. The use of a compound of formula I as defined in claim 5 in which in formula (I) $R^4$ is in the 7-position.

8. The use of a compound of formula I as defined in claim 5 in which in formula (I) $R^3$ is in the 8-position and $R^4$ is in the 7-position.

9. The use of a compound of formula I as defined in claims 5, 6, 7 or 8 in which in formula (I) $R^4$ is $SO_n(C_1$-$C_6)$alkyl; $R^3$ is methoxy or hydroxy; and $R^1$ is hydrogen or methyl.

I0. The use of a compound of formula I as defined in claim 5 in which the compound of formula (I) is 8-hydroxy-7-methylsulfonyl-I-phenyl-2,3,4,5-tetrahydro-IH-3-benzazepine.

II. The use of a compound of formula I as defined in claim 5 in which the compound of formula (I) is selected from one group consisting of:

6-chloro-8-hydroxy-I-phenyl-2,3,4,5-tetrahydro-IH-3-benzazepine,

7-bromo-8-hydroxy-I-phenyl-2,3,4,5-tetrahydro-IH-3-benzazepine,

8-hydroxy-7-methyl-I-phenyl-2,3,4,5-tetrahydro-IH-3-benzazepine,

8-hydroxy-7-methylthio-I-phenyl-2,3,4,5-tetrahydro-IH-3-benzazepine,

8-hydroxy-I-phenyl-7-trifluoromethyl-2,3,4,5-tetrahydro-IH-3-benzazepine,

7-bromo-8-methoxy-3-methyl-I-phenyl-2,3,4,5-tetrahydro-IH-3-benzazepine,

7,8-dichloro-3-methyl-I-phenyl-2,3,4,5-tetrahydro-IH-3-benzazepine,

7,8-dihydroxy-I-phenyl-2,3,4,5-tetrahydro-IH-3-benzazepine,

8-hydroxy-7-methoxymethyl-I-phenyl-2,3,4,5-tetrahydro-IH-3-benzazepine,

8-hydroxy-7-hydroxymethyl-I-phenyl-2,3,4,5-tetrahydro-IH-3-benzazepine,

7-hydroxy-8-methanesulfonamido-I-phenyl-2,3,4,5-tetrahydro-IH-3-benzazepine,

7-acetamido-8-hydroxy-I-phenyl-2,3,4,5-tetrahydro-IH-3-benzazepine,

7-hydroxy-8-methylsulfonyl-I-phenyl-2,3,4,5-tetrahydro-IH-3-benzazepine,

8-methoxy-7-methylsulfonyl-I-phenyl-2,3,4,5-tetrahydro-IH-3-benzazepine, or

8-hydroxy-I-(4-hydroxyphenyl)-7-methyl-2,3,4,5-tetrahydro-IH-3-benzazepine,

in the manufacture of a medicament for the treatment of gastrointestinal motility disorders.

I2. The use of a compound of formula I as defined in claim 5 in the manufacture of a medicament for the treatment of gastroesophageal reflux disease or delayed gastric emptying.

I3. The use of of a compound selected from the group consisting of:

8-hydroxy-7-methylsulfonyl-I-phenyl-2,3,4,5-tetrahydro-IH-3-benzazepine.

7-hydroxy-8-methylsulfonyl-I-phenyl-2,3,4,5-tetrahydro-IH-3-benzazepine,

8-methoxy-7-methylsulfonyl-I-phenyl-2,3,4,5-tetrahydro-IH-3-benzazepine, or

8-hydroxy-I-(4-hydroxyphenyl)-7-methyl-2,3,4,5-tetrahydro-IH-3-benzazepine,

in the manufacture of a medicament for the treatment of gastroesophageal reflux disease or delayed gastric emptying.

Claims for the following Contracting States: AT, GR and ES.

I. A process for preparing a pharmaceutical composition which comprises bringing into association a compound of formula II

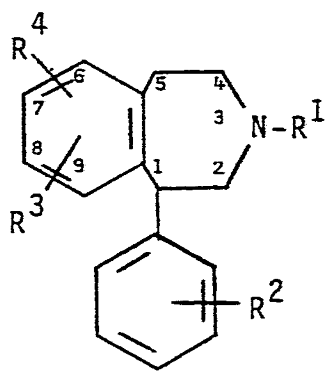

(II)

in which:

$R^1$ is hydrogen, $C_1$-$C_6$-alkyl or $C_3$-$C_5$alkenyl;

$R^2$ is hydrogen, hydroxy, $C_1$-$C_6$alkoxy, halogen, trifluoromethyl, $C_1$-$C_6$alkyl, $SO_n$ ($C_1$-$C_6$)alkyl, $SO_nCF_3$, $SO_n$phenyl or $SO_2NR^5R^6$;

$R^3$ is hydrogen, halogen, $CF_3$, $C_1$-$C_6$alkyl or $R^7O$;

$R^4$ is SO($C_1$-$C_6$)alkyl, $SO_2$($C_1$-$C_6$)alkyl, $C_2$-$C_6$alkylsulfonamido, formamido, $C_3$-$C_6$alkanoylamino, hydroxymethyl or $C_1$-$C_6$alkoxymethyl;;

n is O, I or 2;

$R^5$ and $R^6$ are hydrogen or $C_1$-$C_6$alkyl; and

$R^7$ is hydrogen, $C_1$-$C_6$alkyl or $C_1$-$C_6$alkanoyl;

or a pharmaceutically acceptable acid addition salt thereof; and a pharmaceutically acceptable carrier.

2. A process according to claim I in which the compound of formula II is 8-hydroxy-7-methylsulfonyl-I-phenyl-2,3,4,5-tetrahydro-IH-3-benzazepine.

3. A process according to claim I in which the compound of formula II is:

7-hydroxy-8-methylsulfonyl-l-phenyl-2,3,4,5-tetrahydro-IH-3-benzazepine,

8-methoxy-7-methylsulfonyl-l-phenyl-2,3,4,5-tetrahydro-IH-3-benzazepine,

8-hydroxy-7-methoxymethyl-l-phenyl-2,3,4,5-tetrahydro-IH-3-benzazepine, or

8-hydroxy-7-hydroxymethyl-l-phenyl-2,3,4,5-tetrahydro-IH-3-benzazepine.

4. the use of a compound of formula (I)

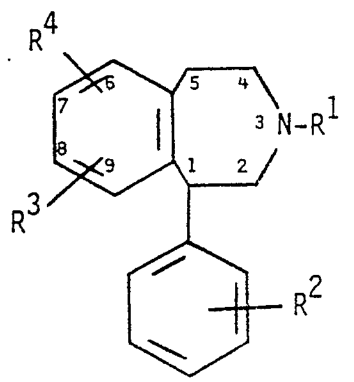

(I)

in which:

$R^1$ is hydrogen, $C_1$-$C_6$alkyl or $C_3$-$C_5$alkenyl;

$R^2$ is hydrogen, hydroxy, $C_1$-$C_6$alkoxy, halogen, trifluoromethyl, $C_1$-$C_6$alkyl, $SO_n$ ($C_1$-$C_6$)alkyl, $SO_nCF_3$, $SO_n$phenyl or $SO_2NR^5R^6$;

$R^3$ is hydrogen, halogen, $CF_3$, $C_1$-$C_6$alkyl or $R^7O$;

$R^4$ is hydroxy, $C_1$-$C_6$alkoxy, $C_1$-$C_6$alkyl, $SO_n(C_1$-$C_6)$ alkyl, $NH_2$, $NO_2$, halogen, trifluoromethyl, $C_1$-$C_6$alkylsulfonamido, $C_1$-$C_6$alkanoylamino, hydroxymethyl or $C_1$-$C_6$alkoxymethyl, except that when $R^3$ is hydroxy in the 7-position and $R^1$ is hydrogen or when $R^3$ is hydroxy in the 8-position and $R^1$ is methyl, $R^4$ may be halogen or trifluoromethyl only if it is in the 6-or 9-position;

n is 0, I or 2;

$R^5$ and $R^6$ are hydrogen or $C_1$-$C_6$alkyl; and

$R^7$ is hydrogen, $C_1$-$C_6$alkyl or $C_1$-$C_6$alkanoyl,

or a pharmaceutically acceptable acid addition salt thereof; in the manufacture of a medicament for the treatment of gastrointestinal motility disorders.

5. The use of a compound of formula I as defined in claim 4 in which in formula (I) $R^2$ is hydroxy, $SO_2CH_3$, $SOCH_3$, $SO_2NH_2$, $SO_2NHCH_3$ or $SO_2N(CH_3)_2$.

6. The use of a compound of formula I as defined in claim 4 in which in formula (I) $R^4$ is in the 7-position.

7. The use of a compound of formula I as defined in claim 4 in which in formula (I) $R^3$ is in the 8-position and $R^4$ is in the 7-position.

8. The use of a compound of formula I as defined in claims 4, 5, 6 or 7 in which in formula (I) $R^4$ is $SO_n(C_1$-$C_6)$alkyl; $R^3$ is methoxy or hydroxy; and $R^1$ is hydrogen or methyl.

9. The use of a compound of formula I as defined in claim 4 in which the compound of formula (I) is 8-hydroxy-7-methylsulfonyl-l-phenyl-2,3,4,5-tetrahydro-IH-3-benzazepine.

I0. The use of a compound of formula I as defined in claim 4 in which the compound of formula (I) is selected from the group consisting of:

6-chloro-8-hydroxy-l-phenyl-2,3,4,5-tetrahydro-IH-3-benzazepine,

7-bromo-8-hydroxy-l-phenyl-2,3,4,5-tetrahydro-IH-3-benzazepine,

8-hydroxy-7-methyl-l-phenyl-2,3,4,5-tetrahydro-IH-3-benzazepine,

8-hydroxy-7-methylthio-l-phenyl-2,3,4,5-tetrahydro-lH-3-benzazepine,

8-hydroxy-l-phenyl-7-trifluoromethyl-2,3,4,5-tetrahydro-lH-3-benzazepine,

7-bromo-8-methoxy-3-methyl-l-phenyl-2,3,4,5-tetrahydro-lH-3-benzazepine,

7,8-dichloro-3-methyl-l-phenyl-2,3,4,5-tetrahydro-lH-3-benzazepine,

7,8-dihydroxy-l-phenyl-2,3,4,5-tetrahydro-lH-3-benzazepine,

8-hydroxy-7-methoxymethyl-l-phenyl-2,3,4,5-tetrahydro-lH-3-benzazepine,

8-hydroxy-7-hydroxymethyl-l-phenyl-2,3,4,5-tetrahydro-lH-3-benzazepine,

7-hydroxy-8-methanesulfonamido-l-phenyl-2,3,4,5-tetrahydro-lH-3-benzazepine,

7-acetamido-8-hydroxy-2-phenyl-2,3,4,5-tetrahydro-lH-3-benzazepine,

7-hydroxy-8-methylsulfonyl-l-phenyl-2,3,4,5-tetrahydro-lH-3-benzazepine,

8-methoxy-7-methylsulfonyl-l-phenyl-2,3,4,5-tetrahydro-lH-3-benzazepine, or

8-hydroxy-l-(4-hydroxyphenyl)-7-methyl-2,3,4,5-tetrahydro-lH-3-benzazepine,

in the manufacture of a medicament for the treatment of gastrointestinal motility disorders.

ll. The use of a compound of formula I as defined in claim 4 for the manufacture of a medicament for the treatment of gastroesophageal reflux disease or delayed gastric emptying.

l2. The use of of a compound selected from the group consisting of:

8-hydroxy-7-methylsulfonyl-l-phenyl-2,3,4,5-tetrahydro-lH-3-benzazepine,

7-hydroxy-8-methylsulfonyl-l-phenyl-2,3,4,5-tetrahydro-lH-3-benzazepine,

8-methoxy-7-methylsulfonyl-l-phenyl-2,3,4,5-tetrahydro-lH-3-benzazepine, or

8-hydroxy-l-(4-hydroxyphenyl)-7-methyl-2,3,4,5-tetrahydro-lH-3-benzazepine,

in the manufacture of a medicament for the treatment of gastroesophageal reflux disease or delayed gastric emptying.